# EUROPEAN PATENT APPLICATION

(11) **EP 1 338 638 A1**
(43) Date of publication of application: **27.08.2003**
(21) Application number: 01967701.2
(22) Date of filing: 17.09.2001
(51) Int. Cl.: C09K 15/06, A61K 47/48, A61K 47/10, A61K 7/00, A61K 7/48, C07H 3/04, C11B 5/00

(54) **AGGREGATE-FORMING AGENT**

(30) Priority: 19.09.2000 JP 2000284264; 20.09.2000 JP 2000285577
(71) Applicant: Kabushiki Kaisha Hayashibara Seibutsu Kagaku Kenkyujo, Okayama-shi, Okayama 700-0907 (JP)
(72) Inventor: OKU, Kazuyuki, c/o Kabushiki Kaisha Hayashibara, Okayama-shi, Okayama 700-0907 (JP); KUBOTA, Michio, c/o Kabushiki Kaisha Hayashibara, Okayama-shi, Okayama 700-0907 (JP); FUKUDA, Shigeharu c/o Kabushiki Kaisha Hayashibara, Okayama-shi, Okayama 700-0907 (JP); MIYAKE, Toshio, c/o Kabushiki Kaisha Hayashibara, Okayama-shi, Okayama 700-0907 (JP)
(74) Representative: Daniels, Jeffrey Nicholas
(86) International application number: JP0108048
(87) International publication number: WO02024832

(57) **Abstract**

The present invention is to provide means of inhibiting chemical changes in unsaturated compounds by radical reactions through inhibition of radical formation in such an unsaturated compound: The present invention attains such an objective by providing an agent which comprises trehalose as an effective ingredient to form an association product between trehalose and an unsaturated compound.

## Description

### TECHNICAL FIELD

The present invention relates to a novel agent which is capable of forming an association product from an unsaturated compound, more particularly, to an agent which contains trehalose as an effective ingredient to form an association product between trehalose and an unsaturated compound.

### BACKGROUND ART

It has been well known that products composed of organic compounds such as oils, fats, dyes and synthesized polymers as predominant ingredients have a tendency of causing undesirable changes in qualities such as generation of unpleasant odor, fading and hardening during their storage. One of major processes for such a change may be chemical reactions of organic compounds which undergo in the absence of catalysts. In the absence of catalysts, the reactivity of organic compounds can be usually evaluated with their instability: One of major factors for instability in organic compounds may be their tendency of forming radicals. Thus it can be said that an organic compound with a higher tendency of forming radicals is much more subject to changes in qualities, in particular, chemical changes as described above. Further it has been said that the tendency of forming radicals would closely relate to the presence of unsaturated bonds within molecules, as well as to the type of unsaturated bonds. If a certain factor triggers radical formation in organic compounds, the resultant radicals successively initiate various reactions with intact molecules and/or oxygen molecules which are both present in the vicinity of the radicals, thus resulting in changes as described above.

Chemical changes in products due to reactions which are successively initiated by radicals (referred to as "radical reaction" hereinafter) would be one of possible problems in the fields of foods, beverages, cosmetics, pharmaceuticals and chemical industrial products because radical formation in organic compounds is easily induced under usual surrounding conditions such as light irradiation and heating. Because of these, the suppression or inhibition of radical reactions would be one of important objectives in order to retain the qualities of products, regardless of the field of industries. In the method which is now most frequently applied in many fields, products or their row materials are added with a substance (usually called as "radical scavenger") capable of reacting with radicals, which have been formed, to lead to chemical changes in the substance per se, thus reducing or even eliminating the reactivity of radicals.

The present inventors however found that a method of inhibiting radicals by adding to products a radical scavenger, which is extensively applied in various fields such as fields of foods and beverages to retain their fresh qualities, inevitably bears the demerit that it hardly stops the progress of radical reactions to some extent because one of major purposes of such a method is to inhibit the progress of reactions by radicals which have been formed. The present inventors also found another demerit that as anticipated from the action principle of such a method, the inhibition by radical scavengers never continues in an endless manner. No one has however noticed these demerits in methods using radical scavengers which are now in practical use, and consequently no effective means of solving these demerits have been studied.

The present invention is entirely different in conception from methods using radical scavengers which are now in practical use with the purpose of retaining fresh qualities of products: The present invention is to solve entirely novel objectives which have been found with a distinct conception by the present inventors. The inhibition of radical formation in a target substance, which is the initial stage of radical reactions, does lead to a fundamental stabilization of substances which are subject to alteration by radical reactions.

### DISCLOSURE OF INVENTION

The present inventors have studied for long the production and uses of trehalose. As a result of the studies, the present inventors found that trehalose has the properties of inhibiting the decomposition of fatty acids in foods and beverages, as well as of inhibiting the formation of volatile aldehydes from fatty acid-containing compositions such as foods and beverages: Based on these findings, the present inventors completed use inventions for trehalose which would be feasible in various fields such as fields of foods, beverages and agents for epidermal use, and disclosed them in the specification of Japanese Patent application No.248,071/99 (Japanese Patent Kokai No.123,194/01). At the time of completion for those inventions, there have however been clarified no detailed mechanisms for the inhibition by trehalose on fatty acid decomposition and volatile aldehyde formation.

Thereafter, the present inventors further studied trehalose with the purpose to clarify the action mechanism for the above described properties of trehalose. As the result, the present inventors found a phenomenon: When linoleic acid, an unsaturated fatty acid with 1,4-pentadien structure as represented by the chemical formula - CH=CH-CH₂-CH=CH- (referred to as "unsaturated fatty acid(s) of 1,4-pentadien type") is chemically changed by radical reactions, it gives a conjugated diene, a molecule with a structure as represented by the chemical formula -CH=CH-CH=CH-, as primary reaction product. In case that trehalose is added to this reaction system, the formation of such a conjugated dien is remarkably reduced dependently on the amount of trehalose. Because of these, the present inventors noticed that trehalose may exhibit against at least unsaturated fatty acids of 1,4-pentadine type an activity of inhibiting the progress of primary stages in radical reactions, in particular, the progress of radical formation due to such a fatty acid.

Thus the present inventors first studied the effects of addition of trehalose on radical formation in linoleic acid and linolenic acid, another unsaturated fatty acid of 1,4-pentadiene type, respectively. As the result, it was confirmed that trehalose much more remarkably inhibited radical formation in these fatty acids than other saccharides. While sucrose did not exhibit the properties of inhibiting radical formation when tested similarly as above for comparison.

It has been said that since unsaturated fatty acids of 1,4-pentadiene type commonly bear within their molecule a reactive methylene group as represented by the chemical formula -CH₂- (usually called as "active methylene group"), which is located between two unsaturated bonds, and the hydrogens on active methylene group are abstracted with a relative ease, the radical formation in such an unsaturated fatty acid would be due to hydrogen abstraction reaction. While as to unsaturated fatty acids other than those of 1,4-pentadiene type, it can be thought that since they bear no active methylene group, the radical formation in such a fatty acid may be different in mechanism from that in unsaturated fatty acids of 1,4-pentadiene type: In this case, hydrogen abstraction, for example, from carbon atoms responsible for unsaturated bonds may be involved in radical formation. These suggest that the inhibition of radical formation in unsaturated fatty acids by trehalose may be specific to those of 1,4-pentadiene type, as well as that trehalose may be not effective against unsaturated fatty acids of different types.

Thus, in order to clarify the molecular mechanism for inhibition of radical formation in fatty acids of 1,4-pentadiene type by trehalose, the present inventors compared the degrees of freedom for particular hydrogen atoms in such an unsaturated fatty acid in the presence and absence of trehalose using nuclear magnetic resonance method. As the result, it was found that the presence of trehalose restricted the degree of freedom for active methylene hydrogens in the unsaturated fatty acid: In other words, trehalose and the unsaturated fatty acid are brought into direct association in such a manner that the degree of freedom for hydrogens bound to active methylene group or active methylene hydrogens is restricted, leading to the conclusion that such an association would be one of factors for the inhibition of radical formation. Also was found that in such an association state, trehalose also restricted the degree of freedom for hydrogens bound to a carbon which is involved in an unsaturated bond in the vicinity of active methylene hydrogens. These results strongly suggest that the inhibition of radical formation by trehalose may be exhibited to unsaturated fatty acids of 1,4-pentadidne type, as well as to those of different types.

In order to obtain an evidence for this speculation, the present inventors carried out an additional experiment similarly as in the above where oleic acid, an unsaturated fatty acid of non 1,4-pentadine type, and sucrose as control were used respectively. As the result, it was confirmed that as anticipated in the above, trehalose was brought into direct association with oleic acid in such a manner that the association restricted the degree of freedom for hydrogens bound to either a carbon atom involved in an unsaturated bond or another carbon atom which was in the vicinity of the former carbon atom. While in case of sucrose, there was observed no restriction on the degree of freedom for hydrogens in oleic acid, suggesting the absence of association. Further there were obtained experimental results indicating that oleic acid in the state of association with trehalose was less liable to form radicals in comparison with intact oleic acid. Based on the above described results, it was concluded that the inhibition of radical formation by trehalose was exhibited through the association of trehalose to either an unsaturated bond or a vicinal site thereof within molecules: Thus trehalose does effectively inhibit radical formation in compounds with any carbon-carbon unsaturated bonds, in other words, unsaturated compounds in general, regardless of the number and type of unsaturated bonds within target molecules, as well as of their whole shapes.

Based on this conclusion, the present inventors further studied in various manners the changes in properties of unsaturated compounds due to association with trehalose using as model compound the above described unsaturated fatty acids. As the result, there was found a phenomenon: Unsaturated fatty acids in the state of association with trehalose give and retain a very stable dispersion in solvents such as water in which intact unsaturated fatty acids hardly disperse. Because of these, it is confirmed that trehalose inhibits both radical formation in unsaturated compounds and alteration by subsequent radical reactions through association with unsaturated compounds, as well as that trehalose has the properties of keeping dispersion states of unsaturated compounds stable. The present invention has been completed with the above described results of independent studies by the present inventors.

As described above, based on the novel finding by the present inventors that trehalose associates with unsaturated compounds at the site of an unsaturated bond and/or its vicinal site within unsaturated compounds, the present invention attains the above described objectives by providing an agent which contains trehalose as an effective ingredient to form an association product between trehalose and an unsaturated compound, as well as uses thereof.

### THE BEST MODE of INVENTION

The present invention relates to an agent which contains trehalose as an effective ingredient to form an association product between trehalose and an unsaturated compound. The term "trehalose" as referred to in the present invention means a disaccharide in which two glucose residues are bound via α,α-linkage each other at their reducing groups. Trehalose preparations which are feasible in the present invention are not restricted to those which have a specific purity, form, characteristic and preparation process, as long as they are capable of forming an association product with one or more of the below mentioned unsaturated compounds.

Trehalose preparations which are feasible in the present invention can be prepared with various processes. With an economical viewpoint, it is preferable to employ a process where a non-reducing sacccharide-forming enzyme and a trehalose-releasing enzyme, which are disclosed in Japanese Patent Kokai No.143,876/95, 213,283/95, 322,883/95, 298,880/95, 66,187/96, 66,188/96, 336,388/96 or 84,586/96 by the same applicant, are allowed to act on a partial starch hydrolyzate. With the use of such a process, one can obtain trehalose from starch, a low-cost material, in a good yield: Examples of commercially-available products which are prepared with such a method are "TREHAROSE OF COSMETIC GRADE", a crystalline trehalose hydrate with a trehalose content of 99% or higher on dry solid basis (d.s.b.), commercialized by Hayashibara Shoji, Incorporated, Okayama, Japan; "TREHA", a crystalline trehalose hydrate with a trehalose content of 98% or higher, d.s.b., commercialized by Hayashibara Shoji, Incorporated, Okayama, Japan; and "TREHASTAR", a trehalose syrup with a trehalose content of 28% or higher, d.s.b., commercialized by Hayashibara Shoji, Incorporated, Okayama, Japan. Trehalose can be also prepared by subjecting maltose to the action of either maltose/trehalose converting enzyme as disclosed, for example, in Japanese Patent Kokai Nos.170,977/95, 263/96 and 149,980/96 by the same applicant, or combination of conventional maltose phosphorylase and trehalose phosphorylase. Anhydrous trehalose can be prepared, for example, by drying a crystalline trehalose hydrate as illustrated in the above at a temperature of 70 to 160°C at usual or a reduced pressure, preferably, at a temperatures of 80 to 100°C at a reduced pressure; and alternatively by placing in a crystallizer a concentrated high trehalose content solution, a moisture content of less than 10%, stirring the solution in the presence of seed crystals at 50 to 160°C, preferably, 80 to 140°C, to prepare a massecuite containing anhydrous crystalline trehalose which is then subjected to, for example, block pulverization, fluidized-bed granulation or spray drying at a relatively high temperature under dehydrated conditions to effect crystallization and pulverization. The trehalose preparations thus obtained are favorably usable in the present invention.

The wording "unsaturated compound(s)" as referred to in the present invention means a hydrocarbon whose carbon chain has a carbon-carbon unsaturated bond (referred to as "unsaturated bond" hereinafter) such as double bond and triple bond, and a derivative where hydrogen atom(s) in such a hydrocarbon is substituted with other element or group. Trehalose, the effective ingredient in the association product-forming agent according to the present invention, associates with any compounds thus defined: Since unsaturated compounds bearing only double bond(s) much remarkably associate with trehalose than those bearing only triple bond(s) when compared them for degree of association, unsaturated compounds bearing double bond(s) are preferable as target compounds to which the association product-forming agent according to the present invention is applied. Particular target compounds to which the association product-forming agent according to the present invention is applied are unsaturated compounds which are grouped into either fatty acids, alcohols, simple lipids, conjugate lipids, terpenes, synthesized polymers or vinyl compounds.

The wording "fatty acid(s)" as referred to in the present invention means any chain compounds and their salts which bear one or two carboxy groups along with an optional branched or cyclic structure and/or a hydroxy group. Fatty acids to which the association product-forming agent according to the present invention can be applied are, usually, those which bear within the molecules three or more carbon atoms including those involved in saturated bonds, in other words, unsaturated fatty acids: Examples of such a fatty acid are those of monoene type which bear one double bond, such as oleic acid, palmitoleic acid, nervonic acid, tsuzuic acid, obtusilic acid and vaccenic acid; those of polyene type which bear two or more double bonds, such as linoleic acid, linolenic acid, arachidonic acid, eicosapentaenoic acid, docosapentaenoic acid, docosahexaenoic acid, prostaglandin, thromboxane and leukotriene; those of acetylene type which bear one or more triple bonds, such as lilic acid, ximenic acid, erythrogenic acid and crepenic acid; and those of polyene dicarboxylic acid type which bear two or more double bonds along with two carboxy groups, such as muconic acid.

The wording "alcohols" as referred to in the present invention means any compounds where hydrogen atom(s) on hydrocarbon chain is substituted with hydroxy group(s), including monohydric alcohols which bear one hydroxy group, and polyhydric alcohols which bear two or more hydroxy groups. Typical alcohols to which the association product-forming agent according to the present invention can be applied are, usually, those which bear within the molecules two or more carbon atoms including those involved in unsaturated bond(s): An example of such an alcohol is oleyl alcohol.

The wording "simple lipid(s)" as referred to in the present invention means organic compounds in general which comprise as constituent atoms carbon, hydrogen and oxygen atoms, and bear within the molecules a hydrocarbon chain corresponding to a fatty acid compound: Typical simple lipids are dehydration condensation products or esters between fatty acid compounds and alcohol compounds, and equivalents thereof. Simple lipids to which the association product-forming agent can be applied are those which bear within the molecules unsaturated bond(s): Examples of such a lipid or ester are decyl oleate and octyldodecyl oleate where the alcohol moieties are of monohydric type; propylene glycol dioleate where the alcohol moiety is of propylene glycol type; monoacyl, diacyl and triacyl glycerols which bear alcohol moieties of glycerol type along with one, two or three unsaturated fatty acid moieties per molecule as illustrated above; fatty acid esters of polyglycerin where the alcohol moieties are of glycerin polymer type and the fatty acid moieties are unsaturated fatty acids as illustrated above; and fatty acid esters of sucrose where the alcohol moieties are sucrose and the fatty acid moieties are unsaturated fatty acids as illustrated above, such as sucrose monooleate, sucrose monolinolate and sucrose dioleate. The wording "oils and fats" as usually referred to in the art are compositions which contain triacyl glycerols as predominant constituents: Oils and fats are grouped into "fatty oils", which are in liquid form at surrounding temperature, and "fats", which are in solid form at surrounding temperature. These can be of course the target compounds in the present invention because they usually contain unsaturated compounds.

The wording "conjugate lipid(s)" as referred to the present invention means organic compounds in general which, like simple lipids as defined in the above, bear within the molecules a hydrocarbon chain corresponding to fatty acid compounds, and comprise as constituent atoms carbon, hydrogen and oxygen atoms along with phosphorous and/or nitrogen atom. Usually, conjugate lipids are roughly grouped into four distinct types, glycerophospholipid, glyceroglycolipid, sphingophospholipid and sphingoglycolipid: In the present invention, the wording "conjugate lipid(s)" includes in addition to these, their derivatives and partially degraded products such as ceramides. Examples of conjugate lipids which can be target compounds for the association product-forming agent according to the present invention are those which bear within the molecules unsaturated bond(s): Particular conjugate lipids are glycerophospholipids such as lecithin or phosphatidylcholine, phosphatidylethanolamine and phosphatidylinositol; glyceroglycolipids such as diacylglycerol which bear within the molecules one or more saccharide residues such as glucosyl and galactosyl groups; sphingophospholipids such as sphingomyelin; and sphingoglycolipids such as cerebroside.

The wording "terpene(s)" as referred to in the present invention means organic compounds in general which bear as constituent units isoprene as represented by the chemical formula CH₂=C(CH₃)CH=CH₂ in a chain or cyclic form. In the present invention, the wording "terpene(s)" also includes conjugate terpenes which are partially composed of isoprene structure. The terpene(s) as defined in the above can be target compounds for the association product-forming agent according to the present invention because they usually bear unsaturated bond(s) within the molecules. Examples of such a terpene are monoterpene, diterpene, triterpene, squalene, tetraterpene, carotenoid and conjugate terpenes such as α-carotene, β-carotene, astaxanthine, kantaxanthine, abscisic acid, vitamin A and vitamin E.

The wording "synthetic polymer(s)" as referred to in the present invention means polymers in general which are synthesized in an organic chemical manner, and roughly grouped into synthetic rubber, thermosetting resin and thermoplastic resin. Synthetic polymers which can be target compounds for the association product-forming agent according to the present invention are those which bear within the molecules unsaturated bond(s). Synthetic rubbers are generally more effective target compounds in the present invention because they bear unsaturated bonds: Examples of such a synthetic rubber are isoprene rubber, butadiene rubber, styrene-butadiene rubber, nitrile rubber and nitrile-isoprene rubber. Examples of thermosetting resin are unsaturated polyester resins.

The wording "vinyl compound(s)" as referred to in the present invention means organic compounds in general which bear either a vinyl group as represented by the chemical formula CH₂=CH-, a vinylidene group as represented by the chemical formula CH₂=C= or a vinylene group as represented by the chemical formula -CH=CH-. Vinyl compounds which can be target compounds for the association product-forming agent according to the present invention are, for example, olefinic hydrocarbons such as ethylene, propylene, butylene and isobutylene; polyenic hydrocarbons such as butadiene and isoprene; acid vinyl esters such as vinyl acetate and vinyl laurate; acrylate such as methyl acrylate and ethyl acrylate; metacrylate such as methyl metacrylate and ethyl metacrylate; vinyl ethers such as lauryl vinyl ether; vinyl chlorides; vinylidene chlorides; styrenes; acrylonitriles; acrylamides; maleic acids; vitamin D; vitamin K; styryl dyes as disclosed in the specification of Japanese Patent Application No.343,211/99 (International Publication WO 01/040382) by the same applicant; pentamethine cyanin dyes of indolenine type as disclosed in the specification of Japanese Patent Application No.31,773/00 (Japanese Patent Kokai No.11329/01) by the same applicant; trimethine cyanin dyes as disclosed in the specification of Japanese Patent Application No.41,001/00 (Japanese Patent Kokai No.212454/02) by the same applicant; trimethine cyanin dyes disclosed in the specification of Japanese Patent Application No.46,570/00 (International Publication WO 01/062853) by the same applicant; styryl dyes of dimethine type as disclosed in the specification of Japanese Patent Application No.143035/00 (International Publication WO 01/019923) by the same applicant; styryl dyes as disclosed in the specification of Japanese Patent Applicant No.203,873/00 (Japanese Patent Kokai No.206061/02) by the same applicant; pentamethine cyanin dyes of asymmetric indolenine type as disclosed in the specification of Japanese Patent Application No.275,764/00 (Japanese Patent Kokai No.323179/01) by the same applicant; trimethine cyanin dyes of asymmetric indolenine type disclosed in the specification of International Patent Application PCT/JP00/02349 (International Publication WO 00/061687) by the same applicant; and Kankoh-so No.101 or "Platonin", Kankoh-so No.201 or "Pionin", Kankoh-so No.301 or "Takanal" and Kankoh-so No. 401 or "Luminex" as listed in "The Japanese Standard of Cosmetic Ingredients", edited by the Society of Japanese Pharmacopoeia, Second edition, Annotation I, published by Yakuji Nippo Limited(1984).

Trehalose directly associates with an unsaturated compound as illustrated above at the site of unsaturated bond and/or its vicinal site. The formation of an association product between trehalose and an unsaturated compound can be confirmed, for example, with a method using nuclear magnetic resonance method as detailed in Examples described below. The following will summarize the method: A particle with a magnetic moment causes a resonance phenomenon (magnetic resonance) when irradiated with an alternating magnetic field or an electromagnetic wave in a static magnetic field. Nuclear spin resonance due to spin in atomic nucleus is particularly called as "nuclear magnetic resonance (NMR)". The method by which the absorption of electronic magnetic wave accompanying with such a nuclear magnetic resonance is observed is called as "nuclear magnetic resonance method (arbitrarily abbreviated "NMR" hereinafter). If an external magnetic field irradiated to the magnetic moment system in a particle which is in a thermal equilibrium is suddenly changed, it will lead to a time delay because the system does not immediately come to a new thermal equilibrium. This phenomenon is called as "magnetic relaxation". Since the time for magnetic relaxation or relaxation time is closely related to absorption signals obtained with nuclear magnetic resonance method, the relaxation time can be determined by analyzing absorption signals. Since relaxation time can be deemed to be a merkmal for degree of freedom in atoms, it can be concluded that a bigger degree of freedom leads to a longer relaxation time. Based on this principle, by first determining relaxation times for all hydrogen atoms in an unsaturated compound in an appropriate solvent with or without trehalose, then comparing the obtained relaxation times, it can be confirmed that the presence of trehalose remarkably shortens the relaxation time for hydrogens which are bound to either a carbon atom involved in an unsaturated bond, and those bound to another carbon atom in the vicinity of the former carbon atom. This result indicates that when trehalose and an unsaturated compound are present together, they form an association product in such a manner that trehalose restricts the degree of freedom for hydrogens at the site of an unsaturated bond nd/or its vicinal site in the unsaturated compound.

Such an association between trehalose and an unsaturated compound leads to the stabilization of unsaturated compounds. The wording "stabilization of unsaturated compound(s)" as referred to in the present invention usually means the inhibition of radical formation in unsaturated compounds: Such an inhibition is usually attained in an unsaturated compound which has associated with trehalose through the restriction in degree of freedom for hydrogen atoms bound to either a carbon atom involved in an unsaturated bond, and/or those bound to another carbon atom which is in the vicinity of the former carbon atom. In such a stabilized unsaturated compound, various chemical changes in intact unsaturated compounds which may be induced by radical formation in unsaturated compounds, for example, peroxidation, hydroxylation, decomposition, discoloration, fading, hardening, generation of odor, generation of heat, burning, combustion and polymerization are remarkably inhibited. Such radical formation is not restricted to those which may be induced by specific causes: It has been known that radical formation is usually initiated, for example, by light irradiation or heating in the absence of catalysts, or by the action of either a catalyst such as metal catalyst or other radical such as activated oxygen. Trehalose remarkably inhibits all of these. Because of these properties of trehalose, the association product-forming agent according to the present invention remarkably exhibits the activity of remarkably inhibiting chemical reactions which are deemed to be the same as those initiated by radical formation in unsaturated compounds with the viewpoint of organic chemistry, for example, a reaction which proceeds through the abstraction of hydrogen atoms from unsaturated compounds by the action of a microorganism or enzyme. Dependently on the type of target unsaturated compounds and solvents, the association of unsaturated compounds with trehalose may lead to the stabilization of dispersion state for unsaturated compounds in solvents. This phenomenon more remarkably appears when target unsaturated compounds are insoluble or hardly soluble in water, and water is used as solvent: In case of dispersing such an unsaturated compound into, for example, an oil-in-water or water-in-oil emulsion form, the coexistence of trehalose in such a system keeps it's dispersion state stable. Because of these, the association product-forming agent according to the present invention would be favorably usable in a variety of fields as illustrated below as an agent to stabilize unsaturated compounds, an agent to restrict the degree of freedom for hydrogens bound to a carbon atom involved in an unsaturated bond and/or those bound to another carbon atom which is in the vicinity of the former carbon in unsaturated compounds, an agent to inhibit radical formation in unsaturated compounds, or an agent to stabilize the dispersion state for unsaturated compounds.

The association product-forming agent according to the present invention comprises as an effective ingredient trehalose, which has the above described activities, arbitrarily along with other ingredients, for example, antioxidants, emulsifying agents, surface active agents and solvents, as long as they hinder the above described properties of trehalose. Examples of antioxidants which are feasible in the present invention are ascorbic acid and its salts; ascorbic acid derivatives such as ascorbic stearate and saccharide-transferred ascorbic acids; chelating agent such as ethylenediamine tetraacetate and its salts; citric acid and its salts; tocopherol acetates; dibutyl hydroxytoluenes; tocopherols; palmitic acid and its salts; palmitic ascorbiles; sodium pyrosulfites; butyl hydroxyanisoles; propyl gallates; tea catechins; hydroquinones; and ammonia.

Examples of emulsifying agents or surface active agents feasible in the present invention are esters such as sorbitan esters of fatty acids, polyoxyethylene esters of fatty acids, glycerin monostearates of oleophilic type, glycerin monostearates of autoemulsifying type, trioctanic trimethyrolpropanes, polyglycerin esters of fatty acids, sucrose esters of fatty acids, ethers such as polyoxyethylene alkyl ethers, and phospholipids including glycerophospholipid such as lecitin.

Examples of solvents which are feasible in the present invention are water, alcohols, ketones, esters, glycolethers, ethers and hydrocarbons. In case that ingredient(s) which are arbitrarily used are unsaturated compounds, trehalose has the merits that because of its properties, it stabilizes them to improve their stability and much more retain prescribed functions of each ingredient. The association product-forming agent according to the present invention is provided in a liquid form such as solution, syrup, suspension or emulsion, a solid form such as powder, granule or block, a semisolid form such as cream or paste, gel or a combination thereof which contains trehalose in an amount of 1 to 100 weight %, preferably, 20 to 99.9 weight % on the basis of total weight, dependently on the fields to which the agent is applied.

To form an association product between trehalose and an unsaturated compound with the use of the association product-forming agent according to the present invention, the agent is added, mixed, dissolved, sprinkled, sprayed or coated in or to either an unsaturated compound or a composition or article of an agricultural, forestry or marine product which contains an unsaturated compound as target compound, if necessary, while stirring, shaking or heating in such a manner that trehalose is allowed to contact with the unsaturated compound. In case that the target unsaturated compound is insoluble or hardly soluble in water, it is preferable to use an association product-forming agent which contains either a surface active agent or an emulsifier along with a solvent which dissolves trehalose. As to organic solvents which dissolve trehalose, it is preferable to use a lower alcohol, for example, a monohydric or dihydric alcohol usually bearing 5 or less carbon atoms, desirably, 3 or less carbon atoms. As to the amount of the association product-forming agent according to the present invention used to form an association product between trehalose and an unsaturated compound, it is preferable to use it, usually, in an equal amount or more, desirably, 1.5 to 20-fold more, more desirably, 2 to 10-fold more on molar ratio dependently on the type and form of the unsaturated compound when the molecular weight of the target unsaturated compound is 2,000 or less, desirably, 1,500 or less. The unsaturated compound which has been allowed to contact with trehalose forms an association product therewith.

The association product-forming agent, which exhibits the above described activities, and the method to form an association product using such an agent would be very useful in various fields where unsaturated compounds or either compositions or articles which contain unsaturated compounds are used as row material, additive or final product, and the avoidance from chemical changes of unsaturated compounds are needed: Examples of such a field are of foods and beverages, agricultural, forestry and marine products, cosmetics, pharmaceuticals, articles for daily use, chemical industries, dyes, paints, building materials, perfumes, chemical reagents, synthetic fibers, pigments, photo-sensitive dyes and optical storage medium, in addition to row materials and additives which are used in either of the above described fields. The association product-forming agent according to the present invention exhibits the prescribed function and allows trehalose and a target unsaturated compound to form an association product even when the target unsaturated compound is in an isolated form, purified form or a composition form with other ingredient(s), as long as the agent is used in such a manner that trehalose is allowed to contact with the target unsaturated compound. Thus in either of the fields, the association product-forming agent according to the present invention can be applied in a desired step of providing or processing row materials into final products while considering the type, form and formulation of a target unsaturated compound, and the resultant product can be used intact similarly as conventional products without trehalose.

Association products between trehalose and unsaturated compounds which have been formed by allowing the latter compounds to contact with trehalose can be used without further processing, for example, in an isolated form as unsaturated compound products which exhibit their inherent properties and improved stabilities. Examples of methods to isolate the association product according to the present invention are extraction, filtration, concentration, centrifugation, dialysis, fractional precipitation, crystallization, hydrophobic chromatography, gel filtration chromatography and affinity chromatography. Association products thus isolated can be used as a final product, row material or additive similarly as conventional unsaturated compound products without trehalose in any fields where the association product-forming agent would find the above described uses, as well as a research reagent which is directed to further investigate association between trehalose and unsaturated compounds and also its mechanism for stabilization.

The following Examples A will explain in detail the stabilization of unsaturated compounds by association with trehalose, while the following Examples B will illustrate the application of the association product-forming agent according to the present invention.

### Example A

### Stabilization of unsaturated compounds through association with trehalose

### Example A-1

### Formation of association products between trehalose and unsaturated compounds

There were provided oleic acid of reagent grade (18:1(9): the first numeral indicates the number of carbons, the next numeral, the number of unsaturated bond(s), and the numeral in parenthesis, the serial number for the carbon atom which is involved in an unsaturated bond and located most nearly to the carboxy group when counted up from the carboxy group-binding carbon atom as the first carbon atom. The numerals appeared hereinafter will be defined in the same manner), linoleic acid (18:2 (9,12)) and α-linolenic acid (18:3 (9,12,15)) (abbreviated as "linolenic acid" hereinafter), both as unsaturated compounds, and stearic acid as saturated compound. Ten mg of either fatty acid was dissolved in 0.9ml deuterated methanol (deuteration ratio of 99.8%). The resultant solution of unsaturated or saturated fatty acid was added with 0.1ml aliquot of either heavy water (deuteration ratio of 99.9%) or a solution in the same heavy water, which have been prepared to give a concentration for either trehalose (hydrous crystals of a reagent grade) or sucrose (anhydrous crystals of a reagent grade) of 250mg/ml together with 0.1ml trimethylsilan as internal standard to obtain test solutions. Each test solution was placed in an amount of 0.6 ml in a glass tube and then subjected to NMR analysis.

In the NMR analysis, an NMR spectrometer, commercialized by Japan Electronics Company Limited under the Tradename of "Type JNM-AL300", was used, and its observation mode and resonance frequency were set to proton nucleus and 300.4 MHz respectively prior to observation. The spectrometer was set with either test solution in glass tube and operated in accordance with its operation manual, thus determining the spin-lattice relaxation time (abbreviated as "relaxation time" hereinafter) for each hydrogen atom in the unsaturated and saturated compounds under these conditions. Each peak and chemical shift obtained as the results in this analysis were assigned while referring to the data reported in "Report of Project Study on the Development of High-level Purification and Extraction Techniques for DHA", published by the Foundation Japan Fishery Fat Associate, page 63 (1997). Based on the analysis data, relaxation time was determined for each assigned hydrogen atom or proton. The assignment and relaxation time for each hydrogen atom obtained by the above analysis were as shown in Tables 1 to 4.

**Table 1**

| Relaxation time for hydrogen atoms in oleic acid ( 18 : 1 (9) ) | | | | |
|---|---|---|---|---|
| Proton number ∗1 | Chemical sift (ppm) | Relaxation time (second)∗2 | | |
| | | No saccharide | Trehalose | Sucrose |
| 9, 10 | 5.346 | 0.871 | 0.508 (58) | 0.949 (109) |
| | 5.329 | 0.797 | 0.632 (79) | 0.679 (85) |
| 2 | 2.273 | 2.458 | 2.916 (119) | 2.868 (117) |
| 8, 11 | 2.046 | 1.043 | 1.124 (80) | 1.318 (94) |
| 3, 4, 5, 6, 7, 12, 13, 14, 15, 16, 17 | 1.360 | 2.539 | 2.615 (103) | 2.539 (100) |
| 18 | 0.900 | 1.621 | 1.524 (94) | 1.577 (97) |

| | | | | |
|---|---|---|---|---|
| ∗1: "Proton number" is represented with the number of carbon atom to which the proton (or hydrogen) is bound. The number of carbon is serially given when counted up from the carboxy group-bearing carbon as the first carbon atom. The underlined proton is bound to either a carbon atom involved in an unsaturated bond or another carbon which is in the vicinity of a carbon atom involved in an unsaturated bond. | | | | |
| ∗2: Numerals in parentheses are relative values when provided that the relaxation time without saccharide is 100. | | | | |

**Table 2**

| Relaxation time for hydrogen atoms in linoleic acid ( 18 : 2 (9,12) ) | | | | |
|---|---|---|---|---|
| Proton number ∗1 | Chemical sift (ppm) | Relaxation time (second) ∗2 | | |
| | | No saccharide | Trehalose | Sucrose |
| 9, 10, 12, 13 | 5.354 | 2.964 | 1.904 (64) | 2.960 (100) |
| | 5.340 | 2.884 | 1.735 (61) | 2.788 (97) |
| 11 | 2.769 | 1.652 | 0.766 (46) | 1.350 (82) |
| 2 | 2.310 | 1.323 | 1.238 (94) | 1.283 (97) |
| 8,14 | 2.069 | 1.475 | 1.107 (75) | 1.351 (92) |
| 3,4,5,6,7, 15, 16, 17 | 1.385 | 1.311 | 1.258 (96) | 1.228 (94) |
| 18 | 0.903 | 2.995 | 2.938 (98) | 2.799 (93) |

| | | | | |
|---|---|---|---|---|
| ∗1: "Proton number" is represented with the number of carbon atom to which the proton (or hydrogen) is bound. The number of carbon is serially given when counted up from the carboxy group-bearing carbon as the first carbon atom. The underlined proton is bound to either a carbon atom involved in an unsaturated bond or another carbon which is in the vicinity of a carbon atom involved in an unsaturated bond. | | | | |
| ∗2: Numerals in parentheses are relative values when provided that the relaxation time without saccharide is 100. | | | | |

**Table 3**

| Relaxation time for hydrogen atoms in linolenic acid ( 18 : 3 (9, 12, 15)) | | | | |
|---|---|---|---|---|
| Proton number ∗1 | Chemical sift (ppm) | Relaxation time (second) ∗2 | | |
| | | No saccharide | Trehalose | Sucrose |
| 9, 10, 12, 13, 15, 16 | 5.336 | 1.328 | 0.630 (47) | 1.329 (100) |
| | 5.318 | 1.689 | 0.709 (42) | 1.607 (95) |
| 11, 14 | 2.750 | 5.538 | 2.729 (49) | 5.600 (101) |
| 2 | 2.286 | 2.557 | 2.115 (83) | 2.227 (87) |
| 8, 17 | 2.048 | 6.358 | 6.733 (106) | 6.963 (110) |
| 3, 4, 5, 6, 7 | 1.390 | 1.519 | 1.390 (70) | 1.444 (95) |
| 18 | 0.909 | 2.867 | 2.476 (86) | 2.533 (88) |

| | | | | |
|---|---|---|---|---|
| ∗1: "Proton number" is represented with the number of carbon atom to which the proton (or hydrogen) is bound. The number of carbon is serially given when counted up from the carboxy group-bearing carbon as the first carbon atom. The underlined proton is bound to either a carbon atom involved in an unsaturated bond or another carbon which is in the vicinity of a carbon atom involved in an unsaturated bond. | | | | |
| ∗2: Numerals in parentheses are relative values when provided that the relaxation time without saccharide is 100. | | | | |

**Table 4**

| Relaxation time for hydrogen atoms in stearic acid ( 18 ) | | | | |
|---|---|---|---|---|
| Proton numbers ∗1 | Chemical sift (ppm) | Relaxation time (second) ∗2 | | |
| | | No saccharide | Trehalose | Sucrose |
| 2 | 2.279 | 0.930 | 0.874 (94) | 0.900 (97) |
| 3, 4, 5, 6, 7, 8, 9, 10, 11, 12, 13, 14, 15, 16, 17 | 1.332 | 0.878 | 0.813 (93) | 0.821 (94) |
| 18 | 0.904 | 2.224 | 2.079 (93) | 2.122 (95) |

| | | | | |
|---|---|---|---|---|
| ∗1: "Proton number" is represented with the number of carbon atom to which the proton (or hydrogen) is bound. The number of carbon is serially given when counted up from the carboxy group-bearing carbon as the first carbon atom. | | | | |
| ∗2: Numerals in parentheses are relative values when provided that the relaxation time without saccharide is 100. | | | | |

As shown in Tables 1 to 4, in oleic acid, linoleic acid and linolenic acid as unsaturated compounds, the coexistence of trehalose remarkably reduced the relaxation time for hydrogen atoms bound to a carbon atom involved in an unsaturated bond and those bound to another carbon atom which was in the vicinity of the former carbon atom. While in stearic acid as a saturated compound, the coexistence of trehalose did not change the relaxation time for their hydrogen atoms. The use of sucrose did not lead to such a change in relaxation time as observed in the above for the same unsaturated and saturated compounds. These results confirm that trehalose has the properties of directly associating with unsaturated compounds in such a manner that the association restricts the degree of freedom for hydrogens bound to a carbon atom involved in an unsaturated bond and those bound to another carbon atom which is in the vicinity of the former carbon atom.

### Example A-2

### Inhibition of radical formation in unsaturated compounds by trehalose

Unsaturated fatty acids of 1,4-pentadien type are converted into radicals under heating conditions, followed by conversion of their 1,4-pentadine structure into a conjugated dien structure (or formation of a conjugated diene radial). In case that oxygen molecule is present in such a reaction circumstance, oxygen molecule immediately reacts with the resultant conjugated diene radical to form another radical (conjugated diene peroxide radical), followed by the progress of a variety of radical chain reactions. While in case that oxygen molecule is absent in such a reaction circumstance, the resultant conjugated diene radical is not subject to further reactions. Thus the progress of initial radical reaction in such an unsaturated fatty acid can be quantitatively traced by heating the unsaturated fatty acid in the absence of oxygen molecule, and determining the resultant conjugated diene with its ultraviolet absorption due to the conjugated structure. Based on this principle, the following operations were carried out.

One hundred mg of either linoleic acid (18:2 (9,12)) or linolenic acid (18:3 (9,12,15)), 10ml of ethanol and 10ml of 50mM phasphate buffer (pH7.0) which had been deaerated with nitrogen gas were placed in 50ml glass vial, and added with 5ml of 0.5%(w/v), 1%(w/v), 5%(w/v) or 10%(w/v) trehalose solution, 10%(w/v) sucrose solution or deionized water, and the head space in the vial was deaerated with nitrogen gas, followed by sealing its opening with a butyl rubber cap. The vial was then placed in an oven which had been kept at 40°C, and heated at this temperature over a period of 14 days under light-shielding conditions. Thereafter 0.3ml of the mixture in the vial was sampled, admixed with 5ml of 80%(v/v) aqueous ethanol and determined for its absorbance at 233nm using 1cm cell. Before heating, the mixture solution was also determined for absorbance similarly as above immediately after placing each reagent in the vial. The amount of conjugated dienes per gram of linoleic acid or linolenic acid was determined in usual manner with the absorbance data based on the molar absorption coefficient of conjugate diene (27,000). The results were as shown in Table 5.

**Table 5**

| Saccharide | Amount of conjugated dien | | | |
|---|---|---|---|---|
| | Linoleic acid | | Linolenic acid | |
| | mg/g | Relative value | mg/g | Relative value |
| None | 3.67 | 100 | 11.50 | 100 |
| Trehalose 0.5% | 3.06 | 83 | 10.95 | 95 |
| 1% | 2.57 | 70 | 7.80 | 68 |
| 5% | 1.87 | 51 | 6.30 | 55 |
| 10% | 1.28 | 35 | 4.50 | 39 |
| Sucrose 10% | 3.64 | 99 | 12.85 | 112 |

As shown in Table 5, in case that trehalose coexisted, the formation of conjugated dienes was remarkably inhibited dependently on the amount of trehalose. While sucrose exhibited no inhibition even when added to give a concentration which gave the highest inhibition of conjugated dienes in case of using trehalose. These results indicate that trehalose remarkably inhibits changes in such an unsaturated fatty acid of 1,4-pentadine type by radical reactions at the initial stage where the unsaturated fatty acid is converted into radicals. Additional studies where the effects of trehalose and sucrose on the radical formation in oleic acid, an unsaturated fatty acid of different type, were tested with usual methods including spin-trapping method demonstrated that similarly as in the above, only trehalose inhibited radical formation (data not shown in detail).

### Example A-3

### Inhibitory effect of trehalose on chemical changes in unsaturated compounds through radical reactions

One and one half mg of linoleic acid, 2.5 mg of SDS and 25 ml of 60 mM Tris-HCl buffer (pH7.0) were placed in 50 ml glass vial with screw cap, and then added with 0.5ml deionized water along with 1.5 ml of either 25%(w/v) trehalose solution, 25%(w/v) sucrose solution or additional deionized water. The resultant was added with 0.1 ml of 2.5mM hydrogen peroxide and 0.1 ml of 2.5 mM aqueous iron chloride solution as radical initiators, and the vial was sealed. The vial was then placed in an oven which had been kept at 40°C, and heated at this temperature for 24 hours to effect reaction.

After the reaction, 0.5ml of the mixture in the vial was sampled, subjected to methyl esterification and determined for linoleic acid level with conventional method using gas chromatography. Before the reaction, the mixture was also determined for linoleic acid level immediately after placing each reagent in the vial. After the reaction, the rate for residual linoleic acid was evaluated with the obtained data. The results were as shown in Table 6.

**Table 6**

| Saccharide | Amount of linoleic acid | |
|---|---|---|
| | mg/g | Relative value |
| None | 0.04 | 7 |
| Trehalose | 0.33 | 54 |
| Sucrose | 0.11 | 18 |
| (Before reaction) | 0.61 | - |

As shown in Table 6, in the system without saccharide, linoleic acid was reduced to an undetectable level under the conditions in this Example which compulsorily initiated and progressed radical reactions. Such a reduction of linoleic acid was remarkably inhibited when trehalose coexisted in the system. These results indicate that trehalose exhibits the activity of inhibiting radical formation in unsaturated compounds and also subsequent chemical changes due to such a radical reaction even under vigorous conditions which compulsorily initiate and progress radical reactions.

As indicated in Examples A-1 to A-3 while comparing with the results for sucrose, there was obtained a cosistent relationship between the formation of association products with unsaturated compounds, inhibition of radical formation in unsaturated compounds and inhibition of chemical changes in unsaturated compounds through radical reactions. These evidences clearly confirm that trehalose has the properties of associating with unsaturated compounds at the site of an unsaturated site and/or its vicinal site: Such an association rectricts the degree of freedom for hydrogens in unsaturated compounds which are responsible for radical reactions, thus radical formation and chemical changes thereby in unsaturated compounds are inhibited to effect their stabilization. The results in Example A-1 indicate that trehalose associates with saturated compounds in a manner which is different from that for unsaturated compounds. While the specification of Japanese Patent Application No.248,071/99 (Japanese Patent Kokai No.123194/01) by the same applicant discloses that trehalose inhibit the decomposition of both unsaturated and saturated fatty acids. Because of these, it is suggested that trehalose may also have the properties of stabilizing saturated compounds: Such a stabilization may be effected through a mechanism different from those for unsaturated compounds where an association at unsaturated bonds and/or their vicinal sites is involved.

### Example for Reference

### Presence and absence of association between linoleic acid and various saccharides

In accordance with the method in Example A-1, the relaxation time for hydrogen atoms in linoleic acid were determined in the presence of either maltose (hydrous crystals of a reagent grade), neotrehalose (hydrous crystals of a reagent grade) or maltitol (anhydrous crystals of a reagent grade) using NMR method. The results were as shown in Table 7.

**Table 7**

| Relaxation time of hydrogen atoms of linoleic acid ( 18 : 2 (9.12) ) | | | | | |
|---|---|---|---|---|---|
| Proton number∗1 | Chemical sift (ppm) | Relaxation time (second) ∗2 | | | |
| | | No saccharide | Maltose | Neotrehalose | Maltitol |
| 9, 10, 12, 13 | 5.354 5.340 | 2.964 2.884 | 2.475 (84) 2.190 (76) | 2.667 (90) 2.610 (90) | 2.854 (96) 2.789 (97) |
| 11 | 2.769 | 1.652 | 1.197 (72) | 1.035 (63) | 1.240 (75) |
| 2 | 2.310 | 1.323 | 1.285 (97) | 1.352 (102) | 1.450 (110) |
| 8,14 | 2.069 | 1.475 | 1.317 (89) | 1.451 (98) | 1.182 (80) |
| 3, 4, 5, 6, 7, 15, 16, 17 | 1.385 | 1.311 | 1.136 (87) | 1.258 (96) | 1.292 (99) |
| 18 | 0.903 | 2.995 | 2.683 (90) | 2.971 (99) | 2.572 (86) |

| | | | | | |
|---|---|---|---|---|---|
| ∗1: "Proton number" is represented with the number of carbon atom to which the proton (or hydrogen) is bound. The number of carbon is serially given when counted up from the carboxy group-bearing carbon as the first carbon atom. The underlined proton is bound to either a carbon atom involved in an unsaturated bond or another carbon which is in the vicinity of a carbon atom involved in an unsaturated bond. | | | | | |
| ∗2: Numerals in parentheses are relative values when provided that the relaxation time without saccharide is 100. | | | | | |

As shown in Table 7, in case that maltose, neotrehalose or maltitol coexisted with linoleic acid, unlike the case that trehalose was present, there was observed no reduction of relaxation time for hydrogens in linoleic acid. The results for maltose and neotrehalose are consistent with the fact disclosed in the specification of Japanese Patent Application No.248,071/99 (Japanese Patent Kokai No.123194/01) by the same applicant that unlike trehalose, maltose and neotrehalose do not inhibit the decomposition of fatty acids. The specification also discloses that maltitol inhibits the decomposition of both unsaturated and saturated fatty acids as much as trehalose. These facts and results in Table 5 suggest that the inhibition of decomposition in fatty acids by maltitol may be exhibited through a mechanism different from those for trehalose. Because of these, it can be concluded that association with unsaturated compounds, inhibition of radical formation in unsaturated compounds and stabilization of unsaturated compound are very characteristic properties of trehalose which have never been observed in other saccharides.

### Example B-1

### Association product-forming agent

Twenty parts by weight of a crystalline trehalose hydrate, commercialized by Hayashibara Shoji, Incorporated, Okayama, Japan, under the Registered Trademark of "TREHA", 1.5 parts by weight of glycerin fatty acid ester, and 0.1 part by weight of vitamin E were added to 80 parts by weight of water, and sufficiently mixed to homogeneity to obtain an association product-forming agent in a liquid form.

The agent does keep glycerin fatty acid ester and vitamin E stable within the agent: The agent is favorably usable as a stabilizer for unsaturated compounds and their dispersion state in various fields, such as in the field of foods, beverages, cosmetics, pharmaceuticals and chemical industries, where unsaturated compounds or compositions or articles containing the same are used, and the avoidance of their chemical changes is needed.

### Example B-2

### Association product-forming agent

A crystalline trehalose hydrate, commercialized by Hayashibara Shoji, Incorporated, Okayama, Japan, under the Registered Trademark of "TREHA", was dried in vacuo at a temperature of 90°C and a reduced pressure of -300 to -350mmHg over a period of about 7 hours using a jacketed rotary vacuum drier. Thereafter the temperature and pressure were elevated to surrounding levels, and the trehalose powder in the drier was collected to obtain a crystalline powder with an anhydrous crystalline trehalose content of 90%(w/w) or more, d.s.b. One hundred parts by weight of the powder was sufficiently admixed with 0.01 part by weight of butyl hydroxytoluene, thus obtaining an association product-forming agent in a powder form.

The agent is favorably usable as a stabilizer for unsaturated compounds and then in dispersion state in various fields, such as in the field of foods, beverages, cosmetics, pharmaceuticals and chemical industries, where unsaturated compounds or compositions or articles containing the same are used, and the avoidance of their chemical changes is needed.

### Example B-3

### Liquid food for energy supplementation

Thirty-five parts by weight of palm oil, 30 parts by weight of triglyceride with a medium chain length, 15 parts by weight of rapeseed oil, 17 parts by weight of corn oil and 3 parts by weight of labiate oil were admixed to prepare a vegetable oil material. Fifty parts by weight of the vegetable oil material, 20 parts by weight of a crystalline trehalose hydrate, commercialized by Hayashibara Shoji, Incorporated, Okayama, Japan under the Registered Trademark of "TREHA", 1.3 parts by weight of glycerin fatty acid ester, 0.01 parts by weight of vitamin E and 0.02 parts by weight of sodium citrate were admixed with water to give a total amount of 100 parts by weight. One hundred and fifty gram aliquots of the resultant mixture were canned in usual manner.

The product is favorably usable as a food for energy supplement superior in storage stability because it is rich in vegetable oils as energy source, and the stability of the vegetable oils and other unsaturated compound components is improved with the use of trehalose.

### Example B-4

### Powdered oil

One hundred and sixty parts by weight of soybean salad oil and one part by weight of lecithin were admixed at room temperature with 21 parts by weight of water, and the resultant was mixed with 160 parts by weight of the association product-forming agent in Example B-2, pulverized and screened to obtain a powdered oil.

The product is favorably usable in seasoning materials such as mayonnaise and dressing, higher calorie intubation feeding and mixed feeds because stability of the unsaturated compounds in the product are improved by trehalose, and the product also stabilizes unsaturated compounds in various food materials when admixed with them.

### Example B-5

### Edible film

Thirty parts by weight of pullulan, commercialized by Hayashibara Shoji, Incorporated, Okayama, Japan, under the Tradename of "PF-20", 61 parts by weight of water and 8 parts by weight of a trehalose syrup, commercialized by Hayashibara Shoji, Incorporated, Okayama, Japan, under the Registered Trademark of "TREHASTAR", were mixed to homogeneity to obtain an edible adhesive. After adjusting the solid concentration of the adhesive to 15%(w/w), 100 parts by weight of the adhesive was admixed with one part by weight of carrageenan and 0.1 part by weight of lecithin to homogeneity, and the resultant was casted in usual manner on a polyester film, prepared into a film, thickness of 0.03mm, at a peeling rate of 3m/min, and dried by the ventilation of 90°C air to obtain a product.

The product is an edible film which is, unlike those solely using pullulan, not readily soluble but gradually soluble and degradable in an aqueous system, as well as characterized by an excellent storage stability because the stability of unsaturated compounds in the product is improved by trehalose: Thus the product is favorably usable similarly as wafer sheet in the fields of foods, beverages and pharmaceuticals.

### Example B-6

### Cream for epidermal use

Thirty parts by weight of a purified water was admixed with 3 parts by weight of propyleneglycol and 3 parts by weight of a crystalline trehalose hydrate, commercialized by Hayashibara Shoji, Incorporated, Okayama, Japan, under the Tradename of "TREHAROSE OF COSMETIC GRADE", and the resultant was heated up to 70°C to prepare an aqueous layer for cream. While 36 parts by weight of squalene, 9 parts by weight of reducing lanolin, 6 parts by weight of beeswax, 5.5 parts by weight of oleyl alcohol, 4 parts by weight of fatty acid glyceride, 2 parts by weight of monooleic acid polyoxyethylene sorbitan (20 E.O.), 2 parts by weight of lipophilic glycerin monostearate, and appropriate amounts of flavor and antioxidant were mixed to homogeneity at 70°C to prepare an oil layer for cream. The oil layer was placed on the surface of the above described aqueous layer, subjected to a preliminary emulsification in usual manner, emulsified to homogeneity with a homogenizing mixer and cooled to obtain a cream of oil-in-water type for epidermal use.

The product is favorably usable as a high-quality cream for epidermal use with an improved storage stability because it exhibits an excellent moisture-retaining activity on the skin, and the stability of unsaturated compounds in the major moisture-retaining oil ingredients is improved by trehalose.

### Example B-7

### Gel for epidermal use

One part by weight of a carboxyvinyl polymer, commercialized by Wako Pure Chemical Industry Limited, Tokyo, Japan, under the Tradename of "Hiviswako 104", and 1,3-buthylene glycol were added with an appropriate amount of water, and then dispersed in usual manner to homogeneity while stirring. One part by weight a crystalline trehalose hydrate, commercialized by Hayashibara Shoji, Incorporated, Okayama, Japan, under the Tradename of "TREHAROSE OF COSMETIC GRADE", one part by weight of poly(vinylpyrrolidone), 0.003 parts by weight of Kankoso No.101, appropriate amounts of flavor and antioxidant and a purified water were admixed to give a total amount of 100 parts by weight and adjusted to pH7.2 to obtain a gel product for epidermal use.

The product is favorably usable as a gel for epidermal use with an improved storage stability in the treatment and prevention of skin diseases such as frostbite and skin damages because it is superior in extending properties and the stability of Kankoso No.101 is improved by trehalose.

### FEASIBILITY IN INDUSTRIES

As explained above, the present invention is based on an entirely novel finding by the present inventors that trehalose directly associates with unsaturated compounds to stabilize them. The association product-forming agent of the present invention fundamentally stabilizes target compounds through an action mechanism entirely different from those for radical scavengers which have been extensively used to stabilize organic compounds. The feasibility of the association product-forming agent, method to form association products, and association products between trehalose and unsaturated compounds would extend over the fields of foods, beverages, agricultural, forestry and marine products, cosmetics, pharmaceuticals, articles for daily use, chemical industries, dyes, paints, building materials, perfumes, chemical reagents, synthetic fibers, pigments, photo-sensitive dyes and optical storage media: Thus the present invention is very significant in industries.

The present invention, which exhibits such a remarkable effect, is very significant and greatly contributive to the art.

## Claims

1. An agent, which contains trehalose as an effective ingredient to form an association product between trehalose and an unsaturated compound.

2. The association product-forming agent described in claim 1, which is in the form of a stabilizer for an unsaturated compound.

3. The association product-forming agent described in claim 1 or 2, said agent being to restrict the degree of freedom for a hydrogen atom bound to a carbon atom involved in an unsaturated bond, and/or that bound to another carbon atom which is in the vicinity of the former carbon atom within an unsaturated compound.

4. The association product-forming agent described in claim 1, 2 or 3, said agent being to inhibit radical formation in an unsaturated compound.

5. The association product-forming agent described in any one of claims 1 to 4, which is in the form of a stabilizer for dispersion state of an unsaturated compound.

6. The association product-forming agent described in any one of claims 1 to 5, said agent being to form an association product between trehalose and an unsaturated compound which is grouped into either fatty acid, alcohol, simple or conjugated lipid, terpene, synthesized polymer and vinyl compound.

7. The association product-forming agent described in any one of claims 1 to 6, which additionally contains one or more members selected from the group consisting of antioxidant, emulsifier, surface active agent and solvent.

8. A method for forming an association product between trehalose and an unsaturated compound, **characterized by** allowing the trehalose in the association product-forming agent described in any one of claim 1 to 7 to contact with an unsaturated compound.

9. The method for forming an association product described in claim 8, which restricts the degree of freedom for a hydrogen atom bound to a carbon atom which is involved in an unsaturated carbon, and/or that bound to another carbon atom which is in the vicinity of the former carbon atom within the unsaturated compound.

10. The method for forming an association product described in claim 8 or 9, which is to inhibit radical formation in an unsaturated compound.

11. The method for forming an association product described in claim 8, 9 or 10, which is to stabilize the dispersion state of an unsaturated compound.

12. The method for forming an association product described in any one of claims 8 to 11, which allows the trehalose in the association product-forming agent described in any one of claims 1 to 6 to contact with an unsaturated compound which is contained in foods, beverages, cosmetics, pharmaceuticals or materials thereof.

13. An association product between trehalose and an unsaturated compound.

14. The association product described in claim 13, wherein the association site is located at an unsaturated bond and/or its vicinity.

15. A composition, which comprises the association product described in claim 13 or 14.
